# EUROPEAN PATENT SPECIFICATION

(11) **EP 0 975 223 B1**
(45) Date of publication and mention of the grant of the patent: **07.01.2009**
(21) Application number: 98914573.5
(22) Date of filing: 07.04.1998
(51) Int. Cl.: A01N 43/40, A61K 8/49, A61Q 5/02, B01J 13/00, C07F 1/08

(54) **IN-SITU GENERATION OF ZINC PYRITHIONE**
IN-SITU-ENTWICKLUNG VON ZINKPYRITHION
PREPARATION IN SITU DE PYRITHIONE DE ZINC

(30) Priority: 18.04.1997 US 44629; 11.03.1998 US 38026
(43) Date of publication of application: 02.02.2000
(73) Proprietor: Arch Chemicals, Inc., Norwalk, CT 06856-4500 (US)
(72) Inventor: HANI, Rahim, Cheshire, CT 06410 (US); POLSON, George, A., Harwinton, CT 06791 (US)
(74) Representative: Bannerman, David Gardner
(86) International application number: PCT/US1998/006917
(87) International publication number: WO 1998/047372

(56) References cited:
- WO-A-97/01397
- WO-A-97/03637
- US-A- 4 323 683
- US-A- 4 632 991
- US-A- 4 940 578
- US-A- 5 540 860
- US-A- 5 650 095
- DATABASE WPI Derwent Publications Ltd., London, GB; AN 1996-112602 XP002211951 RYUHODO SEIKADU KK: "Shampoo, having very high dispersion stability of zinc pyrithione and good foaming - comprises polyoxyethylene lauryl ether sulphate, lauric acid diethanolamide , cellulose trimethyl ammonium salt and zinc pyrithione" & JP 08 012535 A 16 January 1996 (1996-01-16)
- DATABASE WPI Derwent Publications Ltd., London, GB; AN 1985-059731 XP002211952 KAO CORP: "2-Mercaptopyridine-N-oxide polyvalent metal salt particles prodn.- comprising treating 2-mercaptopyridine-N-oxide monovalent water soluble salt with polyvalent metal salt in the presence of nitrogen compound" & JP 60 016973 A 28 January 1985 (1985-01-28)
- DATABASE CA [Online] CHEMICAL ABSTRACTS SERVICE, COLUMBUS, OHIO, US; ISHIMARU, KATSUTOSHI ET AL: "Preparation of 1-hydroxy-2-pyridinethione salts" retrieved from STN Database accession no. 126:31274 XP002211950 & JP 08 283242 A (YOSHITOMI PHARMACEUTICAL, JAPAN) 29 October 1996 (1996-10-29)

## Description

This invention relates generally to zinc pyrithione-containing personal care compositions, and more particularly to methodology for preparing these compositions using in-situ transchelation of a soluble pyrithione salt with pyrithione acid or a soluble pyrithione salt.

Sodium pyrithione (also called the sodium salt of 1-hydroxy-2-pyridinethione, sodium pyridine-2-thiol-N-oxide, or 2-pyridinethiol-1-oxide, Na salt) has excellent antimicrobial properties, and is a well-known commercial product typically employed as a biocide and preservative in functional fluids, such as metalworking fluids, lubricants, cosmetics and toiletries. Sodium pyrithione is commonly made by reacting 2-chloropyridine-N-oxide with NaSH and NaOH, as disclosed, for example, in U.S. Patent No. 3,159,640.

Likewise, zinc pyrithione [also known as zinc pyridine-2-thiol-N-oxide or bis [1-hydroxy-2(H) pyridinethionato]-zinc] is a broad-spectrum antimicrobial agent and preservative in metalworking fluids, plastics, paints, adhesives and cosmetics. One of the principal applications for zinc pyrithione is its use as an anti-dandruff agent in shampoo.

Zinc pyrithione is typically made at the sodium pyrithione production plant by reacting sodium pyrithione with a zinc salt, such as zinc sulfate, to form a zinc pyrithione precipitate, as disclosed, for example in U.S. Patent No. 2,809,971, which is typically washed and then dispersed in water to form an aqueous dispersion of zinc pyrithione in water. This conversion of sodium pyrithione to form the zinc pyrithione dispersion at the pyrithione manufacturing plant is labor intensive. Moreover, the resulting zinc pyrithione dispersion is subject to settling problems, inasmuch as the zinc pyrithione tends to physically settle out of the dispersion during shipment or storage prior to use of the dispersion. This settling problem necessitates mixing of the dispersion by the manufacturer of personal care compositions containing the dispersion in order to insure homogenity of the dispersion in the composition. In addition, the settling problem can result in "caking" of the zinc pyrithione in the bottom of the drum in which it is stored and shipped, particularly when this product is stored for several months or subjected to wide fluctuations in ambient temperature.

In view of the above, it should be clear that there is a need for improved methodology for preparing zinc pyrithione-containing personal care compositions without risking the zinc pyrithione dispersion settling problems encountered heretofore. The present invention provides one solution to this need.

In one aspect, the present invention relates to a process for preparing a zinc pyrithione-containing personal care composition which comprises reacting, in a personal care composition comprising at least one surfactant and having a higher viscosity than zinc pyrithione in water alone pyrithione acid or a pyrithione salt other than zinc pyrithione, or a combination thereof, with a zinc compound that is soluble in the personal care composition, said zinc compound being selected from the group consisting of zinc salts of organic acids, zinc salts of inorganic acids, zinc hydroxide, zinc oxide, and combinations thereof, said zinc compound being soluble in the personal care composition, thereby causing in-situ transchelation of the pyrithione salt with the zinc salt to form said zinc pyrithione-containing personal care composition wherein the personal care composition contains:
a) from about 5% to about 70% of water or an alcohol;
b) from about 2% to about 50% of at least one dispersant or surfactant; and
c) from about 0.1% to about 2% of particles of the *in situ* prepared zinc pyrithione; based upon the weight of said personal care composition, with the proviso that the total of (a) + (b) + (c) does not exceed 100%.
Pyrithione salts useful as reactants in this process include magnesium pyrithione, strontium pyrithione, copper pyrithione, zinc pyrithione, cadmium pyrithione, zirconium pyrithione, and combinations thereof. Zinc salts useful as reactants in this process include zinc sulfate, zinc chloride, zinc acetate, and combinations thereof.

In another aspect, the present invention relates to a process for preparing a zinc pyrithione-containing personal care composition selected from the group consisting of shampoo, soap, skin care medicament, and combinations thereof, said process comprising contacting water or an alcohol with at least one surfactant and with a transchelation product produced in situ by reacting pyrithione acid or a pyrithione salt that is soluble in the personal care composition, or a combination thereof, with a zinc compound selected from the group consisting of zinc salts of organic acids, zinc salts of inorganic acids, zinc hydroxide, zinc oxide, and combinations thereof, said zinc compound being soluble in the personal care composition , and wherein the personal care composition has a higher viscosity than zinc pyrithione in water alone.

It has now been surprisingly found in accordance with the present invention that the in situ formation of zinc pyrithione, either in a personal care composition or in a precursor component of the personal care composition, provides several advantages over the prior art use of preformed zinc pyrithione in these formulations. First, the present invention overcomes the above-discussed settling problem associated with aqueous dispersions of zinc pyrithione, since the zinc pyrithione is formed in situ in the personal care composition. The in situ-formed zinc pyrithione particles are found to be stable in the personal care composition. These compositions (e.g., liquid, gel or creme shampoos, liquid, gel or creme skin care medicaments, and liquid, gel or creme soaps) typically contain one or more surfactants, and have a relatively high viscosity. This high viscosity and the presence of surfactant(s) insure that the in situ prepared zinc pyrithione is stable against settling in the personal care composition. Second, shipment of a pyrithione solution (e.g., aqueous sodium pyrithione) to the personal care product manufacturer eliminates the likelihood of a freezing/settling problem that exists when shipping zinc pyrithione dispersion to the personal care product manufacturer. Third, in view of the high solubility of sodium pyrithione in water and in alcohol, relative to the much lower solubility of zinc pyrithione in these fluids, the sodium pyrithione can be shipped in higher concentrations in these fluids, as compared to zinc pyrithione. For example, a solution of up to 50% by weight of sodium pyrithione in water is suitably prepared and utilized in the process and composition of the present invention, whereas commercial dispersions of aqueous zinc pyrithione typically require 60% by weight of water, thus allowing for only 40% by weight of zinc pyrithione. This enables the pyrithione manufacturer to ship less water to the personal care composition manufacturer, thus saving on shipping costs.

The reactants employed to prepare zinc pyrithione in situ in the personal care composition, in accordance with the present invention, are suitably selected to provide not only the zinc pyrithione, but also a thickening agent for the personal care composition. By way of illustration, sodium chloride is commonly used as a thickening agent in personal care compositions. By selecting zinc chloride and sodium pyrithione for in-situ transchelation in the personal care composition, or in its precursor, to form the desired zinc pyrithione, sodium chloride is also formed, providing the added bonus of a thickening agent in the composition. Other useful thickening agents that are produced in situ in an analogous fashion include ammonium chloride and sodium sulfate. On the other hand, if it is desired to have no salt, other than zinc pyrithione, incorporated into the personal care composition by virtue of the transchelation reaction, then pyrithione acid and zinc oxide or zinc hydroxide are suitably employed as the transchelation reactants. These reactants produce only zinc pyrithione and water as reaction products.

The zinc compound used as a transchelation reactant in accordance with the present invention is suitably selected from the group consisting of zinc salts of organic acids, zinc salts of inorganic acids, zinc hydroxide, zinc oxide, and combinations thereof, with the proviso that the zinc compound is soluble in the personal care composition. Illustrative zinc salts include zinc chloride, zinc sulfate, zinc acetate, and combinations thereof. Zinc chloride is preferred. The term "soluble in the personal care composition" is intended to denote that the zinc compound is soluble in the personal care composition in an amount of at least 100 ppm, preferably at least 1000 ppm.

The pyrithione reactant used in the process and composition of this invention is suitably a pyrithione salt, such as sodium pyrithione, potassium pyrithione, lithium pyrithione, chitosan pyrithione, magnesium disulfide pyrithione, ammonium pyrithione, combinations thereof, and the like, or pyrithione acid, or a combination thereof. The preferred pyrithione salt is sodium pyrithione.

Although the molar ratio of the pyrithione reactant relative to the zinc compound is not narrowly critical, it is preferred that between 1 and 1.5 moles of zinc compound be employed per 2 moles of pyrithione used. The amount of pyrithione acid or water-soluble salt of pyrithione that is soluble in the transchelation reaction mixture can vary over a wide range. A preferred amount of pyrithione or water-soluble pyrithione salt is from about 1% to about 50% by weight, based upon the total weight of the reaction mixture.

Useful reaction media for the transchelation reaction of the present invention include water, organic solvents, and combinations thereof. Useful organic solvents include alcohols, such as methanol, ethanol, amines such as diethanolamine, ethers, esters, and the like.

Additional materials, such as dispersants, dispersant/surfactant blends, and the like may be added to the reactants either before, or during, the precipitation reaction to prevent agglomeration of the pyrithione salt particles formed by virtue of the reaction, if desired. Alternatively or additionally, a dispersant or other dispersing agent providing dispersing characteristics to the particles of pyrithione salt formed during the transchelation reaction, may be added at the completion of the reaction to prevent particle agglomeration. Exemplary dispersants include sodium salts of polymerized alkyl naphthalene sulfonic acids such as "DARVAN" (R.T. Vanderbilt), "DEMOL N" (Kao Chemicals), "DAXAD 11" (Hampshire Chemicals), or "TAMOL N" (Rohm and Haas).

The dispersant is suitably used alone or in combination with a surfactant. Suitable surfactants include linear alcohol alkoxylates, such as the linear alcohol ethoxylates, ethyoxylated/propoxylated block copolymers, ethyoxylated/propoxylated fatty alcohols, and polyoxyethylene cetyl ethers, and the like. If desired, the alcohol alkoxylate is suitably endcapped with a lower alkyl group, and such a product is commercially available as POLY-TERGENT® SLF-18 available from Olin Corporation.

Useful anionic surfactants, that are suitably employed in the compositions of the present invention, include alkyl diphenylether disulfonates, alkyl phenyl ethoxylated phosphate esters, carboxylated linear alcohol alkoxylates, linear alkyl benzene sulfonic acid, diisobutyl sulfosuccinate, and alkyl sulfonates.

Other useful anionics are polycarboxylated alcohol alkoxylates, preferably those selected from the group consisting of the acids or organic or inorganic salts of the following: polycarboxylated linear alcohol alkoxylates, polycarboxylated branched alcohol alkoxylates, polycarboxylated cyclic alcohol alkoxylates, and combinations thereof.

Illustrative cationic surfactants include alkyl triammonium halide, non-linear alkyl dimethyl halide and alkyl dimethyl benzyl ammonium halide-containing surfactants. Illustrative amphoteric dispersants include polyglycol ether derivatives, ethoxylate oxazolin derivatives, lauramidopropyl betain and lecithin.

As will be appreciated by those skilled in the art, suitable blends can be employed in the process of the present invention based on various combinations of the above-described surfactants. The dispersant or dispersant/surfactant blend is preferably employed in a total amount of between about 0.05 and 10%, more preferably between about 0.1 and 5%, most preferably between about 0.5 and about 1.5% by weight, based on the total weight of the reaction mixture.

The personal care compositions of the present invention suitably also contain thickening agents. Illustrative thickening agents include cellulose 30 derivatives, for example methyl, hydroxyethyl, hydroxypropyl and carboxymethyl cellulose, poly(vinyl alcohol), poly (vinylpyrolidone), poly(ethyleneglycol), salts of poly(acrylic acid) and salts of acrylic acid/acrylamide copolymers.

In order to increase the stability against freezing, electrolytes there may be included in the personal care composition. Suitable electrolytes include monomers, such as 1,2-diols, for example glycol, 1,2-propylene glycol and 1,2-butylene glycol, or polymers thereof, or ethoxylated compounds. For example reaction products of ethylene oxide with long-chain alkanols, amines, alkyd phenols, poly(propyleneglycol), or poly(butylene glycol), or a combination thereof, or the like.

The minimum temperature of film formation may be reduced by adding solvents, such as ethylene glycol, butyl glycol, ethyl glycol acetate, ethyl diglycol acetate, butyl diglycol acetate, benzene or alkylated aromatic hydrocarbons. As defoaming agents there are suitable for example poly(propylene glycol) and polysiloxanes. Optionally other antimicrobial additives can additionally be incorporated into the personal care compositions of the present invention. Illustrative of such other antimicrobial additives are polymyxin E, polymyxin B, lower alkyl esters of para-hydroxybenzoic acid (so-called "parabens"), salts thereof, and combinations thereof. These optional other antimicrobial additives are suitably employed in a weight ration of from 1 to 10,000 to 10,000 to 1, based upon the amount of pyrithione employed in the personal care composition. Useful optional solvents include methylisobutylketone (herein referred to as "MIBK"), xylene, ethyl benzene, methanol, and combinations thereof.

The temperature employed in the transchelation reaction in accordance with the present invention is suitably from about 0°C and about 100°C, preferably from about 20°C and about 90°C. The pressure employed in the reaction is suitably between one and two atmospheres, although higher pressures may be employed if desired. Advantageously, this reaction can be run at room temperature (i.e., 20°C to 25°C), and atmospheric pressure.

The personal care compositions prepared in accordance with the present invention to contain dispersed particles of zinc pyrithione are suitably employed in any of a variety of personal care applications, in a liquid or spreadable solid form, alone or in combination with an inert carrier such as water, liquid hydrocarbons, ethanol, isopropanol, or the like. They can be employed using conventional procedures to control bacteria and fungi on substrates such as the skin or hair, and can be applied in an antimicrobial amount by conventional procedures such as spraying, dipping, drenching impregnation, and the like. On the substrate, as well as in the personal care composition, the particles of zinc pyrithione suitably have a shape selected from the group consisting of platelets, rods, needles, cylinders, cones, ellipsoids, prisms, parallelepipeds, pyramids, cubes, and combinations thereof. These particles of zinc pyrithione are suitably crystals having a configuration selected from triclinic, monoclinic, orthorhombic, tetragonal, cubic, trigonal, hexagonal now have a shape selected from the group consisting of platelets, rods, needles, cylinders, cones, ellipsoids, prisms, parallelepipeds, pyramids, cubes, and combinations thereof.

Illustrative examples of typical shampoo formulations follow: A typical antidandruff shampoo comprises the following:
(a) Water in an amount of 43.8%
(b) Zinc pyrithione in an amount of 3%
(c) Cocamide DEA in an amount of 1%
(d) Triethanolamine lauryl sulfate in an amount of 40%

A typical premium antidandruff shampoo comprises the following:
(a) Deionized water in an amount of 76%
(b) Di(hydrogenated) tallow phthalic acid amide in an amount of 5%
(c) Preservative
(d) Citric acid, 50% aq soln OR sodium hydroxide (50% soln)
(e) Ammonium chloride

A typical antidandruff shampoo with Conditioner is the following:
(a) Deionized water in an amount of 76%
(b) Di(hydrogenated) tallow phthalic acid amide in an amount of 5%
(c) Preservative
(d) Citric acid, 50% aq soln. OR sodium hydroxide (50% soln)
(e) Ammonium chloride

Another typical antidandruff shampoo with conditioner is the following:
(a) Deionized water in an amount of 23.85%
(b) Sodium laureth sulfate in an amount of 30%
(c) Tricetylammonium chloride in an amount of 0.50%
(d) Cocamide MEA in an amount of 1.70%
(e) Preservative in an amount of 0.05%
(f) Citric acid, 25% aqueous solution

An "extra body" antidandruff shampoo is the following:
(a) Deionized water in an amount of 64.6%
(b) Methyl Paraben in an amount of 0.30%
(c) Triethanolamine lauryl sulfate in an amount of 20%
(d) Cocodimonium hydrolyzed animal protein in an amount of 1%
(e) FD&C Blue No. 1
(f) Citric acid, 50% aq soln.

The following examples are intended to illustrate, but in no way limit the scope of, the present invention. Unless otherwise stated, the "parts" and "%" are "parts by weight" and "percent by weight", respectively.

### EXAMPLE 1

### Transchelation of Zinc Chloride with Sodium Pyrithione In-situ in a Shampoo to produce Zinc Pyrithione Particles in Cube Form in the Shampoo

A sample of a commercial shampoo of the two-in-one shampoo-and-conditioner type was gently heated to about 60°C with constant stirring. The shampoo contained the following ingredients in the approximate amounts specified in weight percent based upon the weight of the shampoo: about 40% of water, about 20%-30% of ammonium laureth sulfate surfactant, about 15% of ammonium lauryl sulfate surfactant, glycol distearate, dimethicone, fragrance, ammonium xylenesulfonate, about 3% of cocamide MEA foam stabilizer, from 2% to 4% of cetyl alcohol and stearyl alcohol, about 0.5% of polyquarternium-10 conditioner, about 1% mineral oil lubricant, sodium phosphate, stearyl alcohol, about 0.5% of DMDM hydantoin preservative, about 1% of sodium phosphate and disodium phosphate buffer, sodium chloride thickener, and FD&C yellow no. 10 and FD&C blue no.1.An aqueous solution of zinc chloride was mixed into the heated shampoo. With stirring, an aqueous solution of the sodium salt of 2-mercaptopyridine-N-oxide was added over a period of 30 minutes. The stoichiometry of the reactants was such that there was a slight excess of zinc chloride. The mixture was stirred to ensure homogeneity and cooled to ambient temperature. A sample of the shampoo with in situ prepared zinc salt of 2-mercaptopyridine-N-oxide was analyzed the particle size and shape of the crystals formed by scanning electron microscopy (SEM). The particles were found to be cubes having dimensions of about 5 by 5 by 5 microns.

### EXAMPLE 2

### Transchelation of Zinc Chloride with Sodium Pyrithione In-situ in a Shampoo to produce Zinc Pyrithione Particles in Platelet Form in the Shampoo

32 g of hydroxypropyl methylcellulose suspending agent was mixed in water (198 ml) and heated to 70°C for 30 minutes. Zinc chloride (3.4 g) was added to the above mixture. The sodium salt of 2-mercaptopyridine-N-oxide (18.6 g) was added to the above mixture over a period of 5 min. and stirred at 70°C. In a separate container, melted cocoamide DEA was added a mixture of triethanolamine lauryl sulfate, triethanolamine, FD&C yellow no. 5, FD&C blue no. 1 and ethylene glycol distearate. The two mixtures were stirred together at 1500 rpm and the temperature was slowly brought down to ambient. A portion of the shampoo thus formed was analyzed for size and shape of the in-situ generated zinc 2-mercaptopyridine-N-oxide particles. Scanning electron microscopy of the particles showed that these particles had a platelet shape. The median particle size obtained by laser light scattering showed that the particles were present in the form of platelets having a median particle size of 3 µm, with a distribution of particle sizes in the range of from 0.7 to 10 microns.

## Claims

1. A process for preparing a zinc pyrithione-containing personal care composition that comprises *in situ* prepared zinc pyrithione, which process is **characterised by** reacting, in a personal care composition comprising at least one surfactant and having a higher viscosity than zinc pyrithione in water alone, pyrithione acid or a pyrithione salt other than zinc pyrithione, or a combination thereof, with a zinc compound that is soluble in the personal care composition, said zinc compound being selected from zinc salts of organic acids, zinc salts of inorganic acids, zinc hydroxide, zinc oxide, and combinations thereof, thereby causing *in situ* transchelation of the pyrithione salt with the zinc salt to form said zinc pyrithione-containing personal care composition, wherein the personal care composition contains:
a) from about 5% to about 70% of water or an alcohol;
b) from about 2% to about 50% of at least one dispersant or surfactant; and
c) from about 0.1% to about 2% of particles of the *in situ* prepared zinc pyrithione; based upon the weight of said personal care composition, with the proviso that the total of (a) + (b) + (c) does not exceed 100%.

2. The process of claim 1 **characterised in that** said personal care composition is a shampoo.

3. The process of claim 1 **characterised in that** said personal care composition is a soap.

4. The process of claim 1 **characterised in that** said personal care composition is a skin care medicament.

5. A process of preparing zinc pyrithione-containing personal care composition selected from shampoo, soap, skin care medicament, and combinations thereof, said process being **characterised by** contacting water or an alcohol with at least one surfactant and with a zinc pyrithione transchelation product produced *in situ* by reacting pyrithione acid, or a pyrithione salt that is soluble in the personal care composition, or a combination thereof, with a zinc compound selected from zinc salts of organic acids, zinc salts of inorganic acids, zinc hydroxide, zinc oxide, and combinations thereof, said zinc compound being soluble in the personal care composition, and wherein the personal care composition has a higher viscosity than zinc pyrithione in water alone.

6. The process of claim 1 or 5 **characterised in that** said zinc salt is selected from zinc sulfate, zinc chloride, zinc acetate, and combinations thereof.

7. The process of claim 1 or 5 **characterised in that** said pyrithione salt is selected from magnesium pyrithione, strontium pyrithione, copper pyrithione, zinc pyrithione, cadmium pyrithione, zirconium pyrithione, and combinations thereof.

## Patentansprüche

1. Verfahren zur Herstellung einer Zink-Pyrithion enthaltenden Körperpflegezusammensetzung, die in situ zubereitetes Zink-Pyrithion enthält, **gekennzeichnet durch** das Reagieren, in einer Körperpflegezusammensetzung, die mindestens eine grenzflächenaktive Substanz und eine höhere Viskosität aufweist als Zink-Pyrithion alleine in Wasser, einer Pyrithionsäure oder eines Pyrithion-Salzes, außer Zink-Pyrithion, oder einer Kombination davon mit einer in der Körperpflegezusammensetzung löslichen Zinkverbindung, wobei die Zinkverbindung aus Zinksalzen aus organischen Säuren, Zinksalzen aus anorganischen Säuren, Zinkhydroxiden, Zinkoxiden und Kombinationen davon ausgewählt ist, wodurch eine in situ Transchelation des Pyrithion-Salzes mit dem Zinksalz bewirkt wird, um die Zink-Pyrithion enthaltende Körperpflegezusammensetzung zu bilden, wobei die Körperpflegezusammensetzung enthält:
a) ca. 5% bis ca. 70% Wasser oder Alkohol,
b) ca. 2% bis ca. 50% mindestens eines Dispergiermittels oder einer grenzflächenaktiven Substanz und
c) ca. 0, 1 % bis ca. 2% Partikel des in situ zubereiteten Zink-Pyrithions,
bezogen auf das Gewicht der Körperpflegezusammensetzung, mit der Maßgabe, dass die Summe (a) + (b) + (c) 100% nicht übersteigt.

2. Verfahren nach Anspruch 1, **dadurch gekennzeichnet, dass** die Körperpflegezusammensetzung ein Haarwaschmittel ist.

3. Verfahren nach Anspruch 1, **dadurch gekennzeichnet, dass** die Körperpflegezusammensetzung eine Seife ist.

4. Verfahren nach Anspruch 1, **dadurch gekennzeichnet, dass** die Körperpflegezusammensetzung ein Hautpflegemedikament ist.

5. Verfahren zur Herstellung einer Zink-Pyrithion enthaltenden Körperpflegezusammensetzung, die aus Haarwaschmittel, Seife, Hautpflegemedikament und Kombinationen davon ausgewählt ist, **gekennzeichnet durch** das Kontaktieren von Wasser oder eines Alkohols mit mindestens einer grenzflächenaktiven Substanz und mit einem Zink-Pyrithion-Transchelationsprodukt, das in situ produziert wurde **durch** das Reagieren einer Pyrithionsäure oder eines Pyrithionsalzes, das in der Körperpflegezusammensetzung löslich ist, oder eine Kombination davon mit einer Zinkverbindung, die aus Zinksalzen organischer Säuren, Zinksalzen anorganischer Säuren, Zinkhydroxiden, Zinkoxiden und Kombinationen davon ausgewählt ist, wobei die Zinkverbindung in der Körperpflegezusammensetzung löslich ist und wobei die Körperpflegezusammensetzung eine höhere Viskosität als Zink-Pyrithion in Wasser alleine aufweist.

6. Verfahren nach Anspruch 1 oder 5, **dadurch gekennzeichnet, dass** das Zinksalz aus Zinksulfat, Zinkchlorid, Zinkacetat und Kombinationen davon ausgewählt ist.

7. Verfahren nach Anspruch 1 oder 5, **dadurch gekennzeichnet, dass** das Pyrithion-Salz aus Magnesium-Pyrithion, Strontium-Pyrithion, Kupfer-Pyrithion, Zink-Pyrithion, Kadmium-Pyrithion, Zirkonium-Pyrithion und Kombinationen davon ausgewählt ist.

## Revendications

1. Procédé de préparation d'une composition de soin personnelle contenant de la pyrithione de zinc qui comprend de la pyrithione préparée *in situ,* ledit procédé étant **caractérisé par** une réaction, dans une composition de soin personnelle comprenant au moins un tensio-actif et ayant une viscosité supérieure à la pyrithione de zinc dans de l'eau seule, de l'acide de pyrithione ou un sel de pyrithione autre que la pyrithione de zinc, ou une combinaison de ceux-ci, avec un composé de zinc qui est soluble dans la composition de soin personnelle, ledit composé de zinc étant choisi parmi des sels de zinc d'acides organiques, de sels de zinc d'acides inorganiques, de l'hydroxyde de zinc, de l'oxyde de zinc, et des combinaisons de ceux-ci, en provoquant ainsi une transchélation *in situ* du sel de pyrithione avec le sel de zinc, afin de former ladite composition de soin personnelle contenant de la pyrithione de zinc, dans laquelle la composition de soin personnelle contient :
a) d'environ 5 % à environ 70 % d'eau ou d'un alcool ;
b) d'environ 2 % à environ 50 % d'au moins un agent de dispersion ou tensio-actif ; et
c) d'environ 0,1 % à environ 2 % de particules de pyrithione de zinc préparée *in situ ;* sur la base du poids de ladite composition de soin personnelle, à condition que le total de (a) + (b) + (c) ne dépasse pas 100 %.

2. Procédé selon la revendication 1, **caractérisé en ce que** ladite composition de soin personnelle est un shampooing.

3. Procédé selon la revendication 1, **caractérisé en ce que** ladite composition de soin personnelle est un savon.

4. Procédé selon la revendication 1, **caractérisé en ce que** ladite composition de soin personnelle est un soin de la peau.

5. Procédé de préparation d'une composition de soin personnelle contenant de la pyrithione de zinc, choisie parmi un shampooing, un savon, un soin de la peau, et des combinaisons de ceux-ci, ledit procédé étant **caractérisé par** l'entrée en contact de l'eau ou d'un alcool avec au moins un tensioactif et avec un produit de transchélation de pyrithione de zinc produit *in situ,* en faisant réagir l'acide de pyrithione, ou un sel de pyrithione qui est soluble dans la composition de soin personnelle, ou une combinaison de ceux-ci, avec un composé de zinc choisi parmi des sels de zinc d'acides organiques, des sels de zinc d'acides inorganiques, de l'hydroxyde de zinc, de l'oxyde de zinc, et des combinaisons de ceux-ci, ledit composé de zinc étant soluble dans la composition de soin personnelle, et dans lequel la composition de soin personnelle a une viscosité supérieure à la pyrithione de zinc dans de l'eau seule.

6. Procédé selon la revendication 1 ou 5, **caractérisé en ce que** ledit sel de zinc est choisi parmi le sulfate de zinc, le chlorure de zinc, l'acétate de zinc et des combinaisons de ceux-ci.

7. Procédé selon la revendication 1 ou 5, **caractérisé en ce que** ledit sel de pyrithione est choisi parmi de la pyrithione de magnésium, de la pyrithione de strontium, de la pyrithione de cuivre, de la pyrithione de zinc, de la pyrithione de cadmium, de la pyrithione de zirconium et des combinaisons de ceux-ci.
